# EUROPEAN PATENT APPLICATION

(11) **EP 3 623 816 A1**
(43) Date of publication of application: **18.03.2020**
(21) Application number: 18194086.7
(22) Date of filing: 12.09.2018
(51) Int. Cl.: G01N 33/68, A61K 31/00

(54) **GLX-DERIVED MOLECULE DETECTION**

(71) Applicant: GLX Analytix ApS, 1720 Copenhagen V (DK)
(72) Inventor: Della Valle, Brian William, 1720 Copenhagen V (DK); Hempel, Casper, 2870 Dyssegård (DK)
(74) Representative: AWA Denmark A/S

(57) **Abstract**

The present invention relates to biomarkers associated with immune-mediated inflammatory disease (IMID), particular GLX molecules, and even more particular GLX-related glycosaminglycans (GAGs) and GLX-related proteoglycans (PGs).

## Description

The present invention relates to the diagnosis and/or prognosis of inflammatory diseases. More particularly it concerns measurement of the concentration of biomarkers for the prediction of the risk of developing and/or suffering from inflammatory diseases in a subject. Moreover, the invention relates to a medicament for the treatment or prevention of inflammatory diseases in an individual.

### Background of the invention

An immune-mediated inflammatory disease (IMID) is any of a group of conditions or diseases which are characterized by common inflammatory pathways leading to inflammation, and which may result from, or be triggered by, a dysregulation of the normal immune response. All IMIDs can cause end organ damage, and are associated with increased morbidity and/or mortality.

Inflammation is an important and growing area of biomedical research and health care because inflammation mediates and is the primary driver of many medical disorders and autoimmune diseases such as ankylosing spondylitis, psoriasis, systemic lupus erythematosus psoriatic arthritis, Behcet's disease, rheumatoid arthritis, inflammatory bowel disease (IBD), and allergy, as well as many cardiovascular, neuromuscular, and infectious diseases.

IMID is characterized by immune dysregulation, and one underlying manifestation of this immune dysregulation is the inappropriate activation of inflammatory cytokines, such as IL-12, IL-6 or TNF alpha often combined with a reduced production of anti-inflammatory cytokines, whose actions lead to pathological consequences.

While there is evidence for disease associations, progress is being limited by research being pursued in a disease-specific manner, according to clinical presentation, and often being focused on latter stages of disease. Consequently, the early mechanisms surrounding loss of immune tolerance or early inflammatory instigators have been overlooked.

Patent document WO2009068685 extensively discloses the general principles detection, diagnosis, a high-throughput device, and use of such a device for said purpose.

Despite the advent of proteomics for assessing plasma biomarkers, treatment strategies are challenged by the lack of pathognomonic biomarkers predicting disease severity, early signs of new disease flares, and response-to-treatment.

The prevalence of IMID in Western society is estimated at 5%-7%. Currently, there are no straightforward methods to identify common disease characteristics and thus there continues to be an unmet need for biomarkers usable in predicting the risk of developing an IMID, which potentially could lead to organ failures. Additionally, there is a need for further determining the specific type of inflammatory disease, and on this background initiate targeted and effective treatment courses.

### Summary of the invention

In a first aspect of the invention, one or more of these objects are solved by an *ex vivo* method of diagnosing and/or prognosticating at least one immune-mediated inflammatory disease (IMID) in a subject, said method comprising the steps of
a. performing *in vitro* measurement of the level of one or more biomarkers selected from the group consisting of glycosaminoglycans (GAGs) and proteoglycans (PGs) of the glycocalyx in a first biological sample from the subject;
b. optionally, performing *in vitro* measurement of the level of the one or more biomarkers in a second or further biological samples from the subject obtained at a later time point than said first biological sample.
c. comparing the level of said one or more biomarkers to the level of two or more corresponding references; and
d. using the measured value to evaluate the state of the subject.

In a further embodiment, the state of the subject evaluated in step d. is determined as at risk of developing and/or having IMID, if said level of two or more biomarkers are above the corresponding one or more reference levels, and determined as not at risk of developing or having IMID, if said one or more levels are equal to or below the one or more corresponding reference levels.

In yet another embodiment the level of the one or more biomarkers is significantly greater than the corresponding reference value with a P-value of at least <0.05.

The glycocalyx, also known as the pericellular matrix, is a layer composed of glycoprotein and glycolipid that surrounds the cell membranes of most epithelial animal cells. Generally, the carbohydrate portion are involved in the cell-cell recognition, communication, and intercellular adhesion. Thus, the glycocalyx amongst others function to distinguish between its own healthy cells and diseased cells, or invading organisms.

In blood vessels, the endothelial glycocalyx is located on the apical surface facing the lumen. When vessels are stained with cationic dyes such as Alcian blue, transmission electron microscopy shows a small, irregularly shaped layer extending approximately 50-300 nm into the lumen of a blood vessel.. It is present throughout a diverse range of microvascular beds (capillaries) and macrovessels (arteries and veins). The glycocalyx also consists of a wide range of enzymes and proteins that regulate leukocyte and thrombocyte adherence, since its principal role in the vasculature is to maintain plasma and vessel-wall homeostasis. These enzymes and proteins include: Extracellular superoxide dismutase (SOD3), Antithrombin-III,
Growth factors and Chemokines.

The enzymes and proteins listed above serve to reinforce the glycocalyx barrier against vascular and other diseases. Another main function of the glycocalyx within the vascular endothelium is that it shields the vascular walls from direct exposure to blood flow, while serving as a vascular permeability barrier. Its protective functions are universal throughout the vascular system, Another protective function throughout the cardiovascular system is its ability to affect the filtration of interstitial fluid from capillaries into the interstitial space.

Because the glycocalyx is so prominent throughout the cardiovascular system, disruption to this structure has detrimental effects that can cause disease. Certain stimuli that cause atheroma may lead to enhanced sensitivity of vasculature. Initial dysfunction of the glycocalyx can be caused by hyperglycemia or oxidized low-density lipoproteins (LDLs), which then causes atherothrombosis. In microvasculature, dysfunction of the glycocalyx leads to internal fluid imbalance, and potentially edema. In arterial vascular tissue, glycocalyx disruption causes inflammation and atherothrombosis.

Shedding of the glycocalyx can be triggered by inflammatory stimuli, such as tumor necrosis factor-alpha. Whatever the stimulus is, however, shedding of the glycocalyx leads to a drastic increase in vascular permeability. Vascular walls being permeable is disadvantageous, since that would enable passage of some macromolecules or other harmful antigens.

The glycocalyx, which is located on the apical surface of endothelial cells, is composed of a negatively charged network of proteoglycans, glycoproteins, and glycolipids.

In one embodiment of the invention, the biological sample is a blood sample, such as whole blood, plasma, serum or cerebrospinal fluid.

In a further embodiment of the invention the one or more biomarkers are selected from the group consisting of the glycosaminoglycan (GAG) is selected from one or more of the group consisting of chondroitin sulfate (CS), heparan sulfate (HS), hyaluronic acid (HA), dermatan sulfate (DS), keratin sulfate (KS) and or the proteoglycan (PG) is selected from one or more of the group consisting of syndecans, glypicans, and biglycans.

In yet another embodiment, the syndecan is selected from one or more of the group consisting of syndecan-1, syndecan-2, syndecan-3 and syndecan-4 and/or the glypican is selected from one or more of the group consisting of, glypican-1, glypican-2, glypican-3, glypican-4, glypican-5 and glypican-6 and or, the proteoglycan (PG) is selected from one or more of the group consisting of biglycan, perlecan, mimecan, decorin and versican.

In a particular embodiment of the invention, the biomarkers are a combination Syndecan-3, Chondroitin Sulfate (CS), CD44, Heparan Sulfate (HS), Syndecan-4, Syndecan-1, Glypican-1 and Hyaluronic Acid (HA).

It is contemplated that the immune-mediated inflammatory disease diagnosed or prognosticated according to the invention may be or develop into an autoimmune disease, a neuroinflammatory disease, an organ system disease, or more specifically be or develop into one of the following diseases of table 1 and which could be diagnosed or prognosticated by the method of the invention. The individual diseases could be diagnosed or prognosticated either alone or simultaneously in a single biological sample:

**Table 1: List of inflammatory diseases**

| **Autoimmune** | **Neuroinflammatory** |
|---|---|
| | |
| Achalasia | Stroke |
| Addison's disease | Alzheimer's Disease |
| Adult Still's disease | Parkinson's Disease |
| Agammaglobulinemia | Menigitis |
| Alopecia areata | TBI |
| Amyloidosis | Chronic Traumatic Encephalopathy |
| Ankylosing spondylitis | Epilepsy |
| Anti-GBM/Anti-TBM nephritis | Demyelinating Disease |
| Antiphospholipid syndrome | Motor Neuron Disaese |
| Autoimmune angioedema | Amyotrophic Lateral Sclerosis |
| Autoimmune dysautonomia | Mild Cognitive Impairment |
| Autoimmune encephalomyelitis | Dementia |
| Autoimmune hepatitis | Encephalitis |
| Autoimmune inner ear disease (AIED) | |
| Autoimmune myocarditis | **Other Inflammatory** |
| Autoimmune oophoritis | |
| Autoimmune orchitis | Chronic Kidney Disease |
| Autoimmune pancreatitis | COPD |
| Autoimmune retinopathy | Diabetes |
| Autoimmune urticaria | Type II Diabetes |
| Axonal & neuronal neuropathy (AMAN) | Chronic Liver Disease |
| Baló disease | Cirrohosis |
| Behcet's disease | Pericarditis |
| Benign mucosal pemphigoid | Pancreatitis |
| Bullous pemphigoid | Myocarditis |
| Castleman disease (CD) | Arthritis |
| Celiac disease | Osteoarthritis |
| Chagas disease | Bronchiolitis |
| Chronic inflammatory demyelinating polyneuropathy (CIDP) | Vasculitis |
| Chronic recurrent multifocal osteomyelitis (CRMO) | Retinitis |
| Churg-Strauss Syndrome (CSS) or Eosinophilic Granulomatosis (EGPA) | Dermatitis |
| Cicatricial pemphigoid | Encephalitis |
| Cogan's syndrome | Lymphangitis |
| Cold agglutinin disease | Hepatitis |
| Congenital heart block | Vasculitis |
| Coxsackie myocarditis | Atherosclerosis |
| CREST syndrome | Glomerulonephritis |
| Crohn's disease | Nephritis |
| Dermatitis herpetiformis | Glomerulosclerosis |
| Dermatomyositis | Hepatitis |
| Devic's disease (neuromyelitis optica) | Steatohepatitis |
| Discoid lupus | Endocarditis |
| Dressler's syndrome | Myocarditis |
| Endometriosis | Chorioamnionitis |
| Eosinophilic esophagitis (EoE) | |
| Eosinophilic fasciitis | **Organ System diseases** |
| Erythema nodosum | (Brain) |
| Essential mixed cryoglobulinemia | Mental Disorder |
| Evans syndrome | Depression |
| Fibromyalgia | Psychosis |
| Fibrosing alveolitis | Schizoprenia |
| Giant cell arteritis (temporal arteritis) | Huntington's Disease |
| Giant cell myocarditis | Creutzfeldt-Jakob Disease |
| Glomerulonephritis | Prion Disease |
| Goodpasture's syndrome | |
| Granulomatosis with Polyangiitis | (Cardiovascular) |
| Graves' disease | Heart Disease |
| Guillain-Barre syndrome | Myocardial Infarction |
| Hashimoto's thyroiditis | Coronary Heart Disease |
| Hemolytic anemia | Connective Tissue Disease |
| Henoch-Schonlein purpura (HSP) | |
| Herpes gestationis or pemphigoid gestationis (PG) | (Liver Disease) |
| Hidradenitis Suppurativa (HS) (Acne Inversa) | Fatty Liver |
| Hypogammaglobulinemia | Liver Disease |
| IgA Nephropathy | Non-alcoholic liver disease |
| lgG4-related sclerosing disease | Alcoholic liver disease |
| Immune thrombocytopenic purpura (ITP) | |
| Inclusion body myositis (IBM) | (Kidney Disease) |
| Interstitial cystitis (IC) | Nephropathy |
| Juvenile arthritis | |
| Juvenile diabetes (Type 1 diabetes) | (Lung Disease) |
| Juvenile myositis (JM) | COPD |
| Kawasaki disease | Pulmonary Fibrosis |
| Lambert-Eaton syndrome | Sarcoidosis |
| Leukocytoclastic vasculitis | Tuberous Sclerosis |
| Lichen planus | |
| Lichen sclerosus | (Muscluar Disease) |
| Ligneous conjunctivitis | Myopathy |
| Linear IgA disease (LAD) | Neuromusclar Disease |
| Lupus | Pain |
| Lyme disease chronic | |
| Meniere's disease | (Pregnancy) |
| Microscopic polyangiitis (MPA) | PreEclampsia |
| Mixed connective tissue disease (MCTD) | Placental Infarction |
| Mooren's ulcer | Fibrinoid necrosis |
| Mucha-Habermann disease | |
| Multifocal Motor Neuropathy (MMN) or MMNCB | |
| Multiple sclerosis | |
| Myasthenia gravis | |
| Myositis | |
| Narcolepsy | |
| Neonatal Lupus | |
| Neuromyelitis optica | |
| Neutropenia | |
| Ocular cicatricial pemphigoid | |
| Optic neuritis | |
| Palindromic rheumatism (PR) | |
| PANDAS | |
| Paraneoplastic cerebellar degeneration (PCD) | |
| Paroxysmal nocturnal hemoglobinuria (PNH) | |
| Parry Romberg syndrome | |
| Pars planitis (peripheral uveitis) | |
| Parsonage-Turner syndrome | |
| Pemphigus | |
| Peripheral neuropathy | |
| Perivenous encephalomyelitis | |
| Pernicious anemia (PA) | |
| POEMS syndrome | |
| Polyarteritis nodosa | |
| Polyglandular syndromes type I, II, III | |
| Polymyalgia rheumatica | |
| Polymyositis | |
| Postmyocardial infarction syndrome | |
| Postpericardiotomy syndrome | |
| Primary biliary cirrhosis | |
| Primary sclerosing cholangitis | |
| Progesterone dermatitis | |
| Psoriasis | |
| Psoriatic arthritis | |
| Pure red cell aplasia (PRCA) | |
| Pyoderma gangrenosum | |
| Raynaud's phenomenon | |
| Reactive Arthritis | |
| Reflex sympathetic dystrophy | |
| Relapsing polychondritis | |
| Restless legs syndrome (RLS) | |
| Retroperitoneal fibrosis | |
| Rheumatic fever | |
| Rheumatoid arthritis | |
| Sarcoidosis | |
| Schmidt syndrome | |
| Scleritis | |
| Scleroderma | |
| Sjögren's syndrome | |
| Sperm & testicular autoimmunity | |
| Stiff person syndrome (SPS) | |
| Subacute bacterial endocarditis (SBE) | |
| Susac's syndrome | |
| Sympathetic ophthalmia (SO) | |
| Takayasu's arteritis | |
| Temporal arteritis/Giant cell arteritis | |
| Thrombocytopenic purpura (TTP) | |
| Tolosa-Hunt syndrome (THS) | |
| Transverse myelitis | |
| Type 1 diabetes | |
| Ulcerative colitis (UC) | |
| Undifferentiated connective tissue disease (UCTD) | |
| Uveitis | |
| Vasculitis | |
| Vitiligo | |
| Vogt-Koyanagi-Harada Disease | |
| Wegener's granulomatosis (or Granulomatosis with Polyangiitis (GPA)) | |

The amount of biological sample tested is more than or equal to 0.10, µl such as 0.25 µl, 0.5 µl, 0.75 µl, 1 µl, 1.5 µl, 2 µl, 5 µl or 10 µl. Larger volumes may be used depending on the assay.

In one embodiment of the *ex vivo* method according to the invention, step a) is performing *in vitro* measurement of the level of two, three, four, five, ten, twenty or more biomarkers selected from the group consisting of glycosaminoglycans (GAGs) and proteoglycans (PGs) of the glycocalyx.
The platform used to identify the presence of one or more biomarkers.

In another aspect of the invention the objects are solved by providing a substance for use in a method of treating condition in an individual known to have at least one or more biomarkers selected from the group consisting of glycosaminoglycans (GAGs) and proteoglycans (PGs) of the glycocalyx, wherein the biomarkers are at level above the corresponding one or more reference levels, wherein the substance and condition are selected from:

| **Drug** | **IMID** |
|---|---|
| Biologics | Autoimmune Disease |
| Non-steroidal anti-inflammatory drugs | |
| Glucocorticoids | |
| Disease-modifying antirheumatic drugs | |
| Biosimilars | |
| Vasodilators | Stroke |
| Statins | |
| Anticonvulsants | |
| ACE Inhibitors | |
| Cholinesterase inhibitors | Alzheimer's Disease |
| Levodopa | Parkinson's Disease |
| Carbidopa | |
| Carbidopa-levodopa | |
| Dopamine agonists | |
| MAO B inhibitors | |
| Catechol O-methyltransferase (COMT) inhibitors | |
| Anticholinergics | |
| Amantadine | |

Furthermore, the objects are solved by providing a substance for use in a method of treating condition in an individual known to have at least one or more biomarkers selected from the group consisting of glycosaminoglycans (GAGs) and proteoglycans (PGs) of the glycocalyx, wherein the substance and condition are selected from:

| **Substance:** | **Condition (IMID):** |
|---|---|
| Immunosuppressant Drugs | **Lupus** |
| Cyclophosphamide | |
| Azathioprine | |
| Hydroxychloroquine | |
| Methotrexate | |
| Ibuprofen | |
| Naproxen | |
| Sulindac | |
| misoprostol | |
| Prednisolone | |
| methylprednisolone | |
| butesonide | |
| chloroquine | |
| dapsone | |
| chlorambucil | |
| mycophenolate mofetil | |
| Belimumab | |
| Rituximab | |
| dehydroepiandrosterone | |
| Immunosuppressant Drugs | **Rheumatoid Arthritis** |
| Cyclophosphamide | |
| Azathioprine | |
| Belimumab | |
| Hydroxychloroquine | |
| Rituximab | |
| Ibuprofen | |
| Etanercept | |
| methotrexate | |
| sulfasalazine | |
| leflunomide | |
| Infliximab | |
| prednisolone | |
| methylprednisolone | |
| butesonide | |
| Immunosuppressant Drugs | **Inflammatory Bowel Disease** |
| Mesalazine | |
| prednisolone | |
| methylprednisolone | |
| butesonide | |
| Azathioprine | |
| methotrexate | |
| cyclosporin | |
| tacrolimus | |
| adalimumab | |
| Infliximab | |
| certolizumab pegol | |
| Ornidazole | |
| Metronidazole | |
| clarithromycin | |
| rifaximin ciprofloxacin | |
| infliximab | **Celiac Disease** |
| infliximab-abda | |
| infliximab-dyyb | |
| balsalazide | **Crohn's Disease** |
| Mesalamine | |
| olsalazine | |
| Sulfasalazine | |
| Tacrolimus | |
| prednisolone | |
| methylprednisolone | |
| Butesonide | |
| Azathioprine | |
| methotrexate | |
| Cyclosporin | |
| Golimumab | |
| adalimumab-adbm | |
| Adalimumab | |
| Infliximab | |
| infliximab-abda | |
| infliximab-dyyb | |
| vedolizumab | |
| Immunosuppressant Drugs | |
| balsalazide | **Ulcerative Colitis** |
| Mesalamine | |
| olsalazine | |
| Sulfasalazine | |
| prednisolone | |
| methylprednisolone | |
| Butesonide | |
| Azathioprine | |
| methotrexate | |
| cyclosporin | |
| golimumab | |
| adalimumab-adbm | |
| adalimumab | |
| infliximab | |
| infliximab-abda | |
| infliximab-dyyb | |
| vedolizumab | |
| Immunosuppressant Drugs | |
| Calcipotriene | **Psoriasis** |
| Acitretin | |
| Methoxsalen | |
| trioxsalen | |
| adalimumab | |
| Etanercept | |
| infliximab | |
| secukinumab | |
| ixekizumab | |
| apremilast | |
| methotrexate | |
| cyclosporin | |
| Immunosuppressant Drugs | |
| atorvastatin | **Stroke** |
| fluvastatin | |
| lovastatin | |
| pitavastatin | |
| pravastatin | |
| rosuvastatin | |
| simvastatin | |
| Coumadin | |
| Jantoven | |
| Marfarin | |
| clopidogrel | |
| Tissue plasminogen activator | |
| angiotensin-converting enzyme inhibitors | |
| beta-blockers | |
| calcium channel blockers | |
| galantamine | **Alzheimer's Disease** |
| donepezil | |
| rivastigmine | |
| memantine | |
| citalopram | |
| mirtazapine | |
| sertraline | |
| bupropion | |
| duloxetine | |
| imipramine | |

In a particular embodiment, the substance selected from the group composed of atorvastatin, fluvastatin, lovastatin, pitavastatin, pravastatin, rosuvastatin, simvastatin, Coumadin, Jantoven, Marfarin, clopidogrel, Tissue plasminogen activator, angiotensin-converting enzyme inhibitors, beta-blockers, calcium channel blockers for use in a method of treating a stroke in an individual known to have at least one or more biomarkers selected from the group consisting of glycosaminoglycans (GAGs) and proteoglycans (PGs) of the glycocalyx.

In another aspect of the invention, the above-stated objects are solved by providing a kit comprising
- binding agents for at least two biomarkers selected from the group consisting of glycosaminoglycans (GAGs) and proteoglycans (PGs) of the glycocalyx; and
- optionally instructions for using the binding agents in the evaluation of IMID.

Further, the may kit comprise
- binding agents for Syndecan-3, Chondroitin Sulfate (CS), CD44, Heparan Sulfate (HS), Syndecan-4, Syndecan-1, Glypican-1 and Hyaluronic Acid (HA), respectively; and-instructions for using the binding agents in the evaluation of stroke.

In yet another aspect, a microfluidic detection chip for the detection of an IMID infection in a patient is provided comprising
a. a plurality of layers in which a plurality of channels are disposed;
b. a sample input channel into which a biological sample is introduced;
c. one or more separation channels having one or more three-dimensional (3D) separation zones; and
d. one or more channels having one or more optical zones,
wherein the one or more separation channels are coated with one or more biomarkers selected from the group consisting of glycosaminoglycans (GAGs) and proteoglycans (PGs) of the glycocalyx.

In one embodiment, the kit according to the invention further comprises said microfluidic chip wherein the one or more separation channels are coated with said binding agents.

### Brief description of figures

The invention will be described in more detail below by means of non limiting example of embodiments and with reference to the figures, in which:

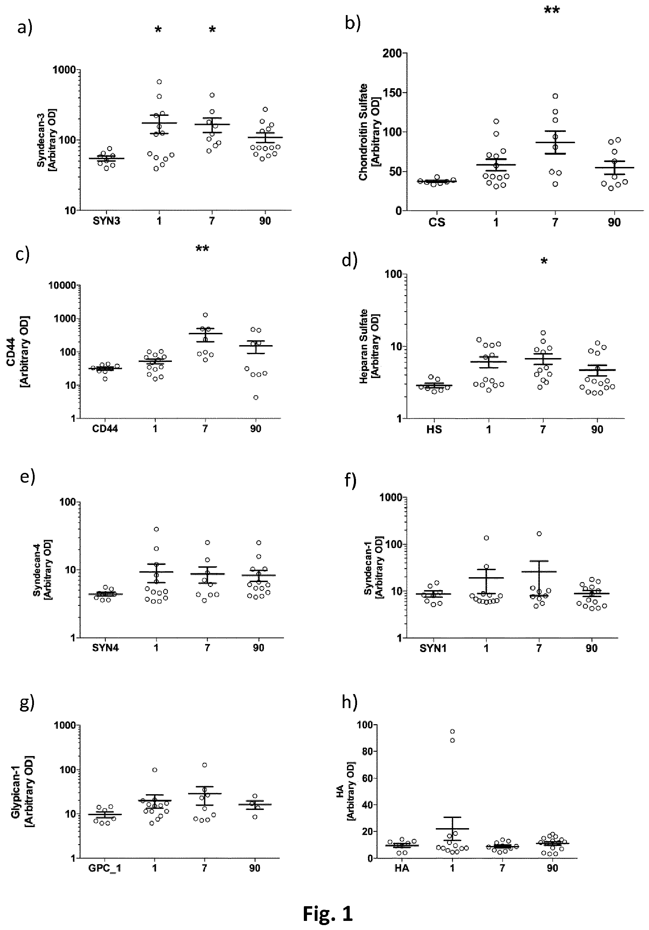

Fig. 1 shows the results of an dot blotting analysis of carbohydrate-associated blood biomarkers for stroke. The biomarker tested are a) Syndecan-3, b) Chondroitin Sulfates (CS), c) CD44, d) Heparan Sulfates (HS), e) Syndecan-4, f) Syndecan-1, g) Glypican-1 and h) Hyaluronic Acids (HA).

### Detailed description

In the following, embodiments of the invention will be described in further detail. Each specific variation of the features can be applied to other embodiments of the invention unless specifically stated otherwise.

The biomarkers of the current invention are carbohydrates found in the thick matrix lining of blood vessels termed the endothelial glycocalyx (GLX). This layer maintains vascular homeostasis and multiple disruptive stimuli leads to shedding of soluble and detectable components and thus loss of this layer. This translates into increased plasma values and we have developed a very sensitive assay using only minute amounts of blood for detecting these changes. These biomarkers may predict risk of or diagnose diseases of the vasculature and brain e.g. multiple sclerosis (MS), lupus, arthritis, inflammatory bowel disease, vasculitis, Alzheimer's disease, generalized dementia, Parkinson's Disease and ALS. There are a wide-range of diseases that may benefit from such an invention inclusive of many rare diseases of specific classes such as but not limited to autoimmune diseases, chronic inflammatory diseases, neurological disease, and cardiovascular disease. The specific diseases are listed in table 2.

The GLX comprises a combination of different proteoglycans and/or glycosaminoglycans. The combinations of these molecules, as detected in biological fluids or samples, are different in different types of diseases, e.g. MS vs Stroke. In this manner, different diseases, present in different organ systems, will be able to predict disease course, predict attacks, and even diagnose disease in complement with other techniques such as MRI.

It is contemplated that GLX cleaving enzymes are activated during immune invasion into diseased organs or alternatively, by auto-activated immune cells destined to cause disease. Indeed, GLX removing enzymes are increased in the blood of a number of diseases, such as multiple sclerosis.

It is further contemplated that a composite biomarker for GLX shedding would improve personalized approaches to disease monitoring and design therapies on a precision medicine principle. Thus, the identification of the entire structure of GLX shedding components in the blood, and the integration of these markers into a composite biomarker allows for the detection of disease, severity and a treatment response.

It is contemplated that a mechanism of action whereby an immune cell arrives at an endothelial cell, secretes enzymes to remove the GLX structure, transmigrates into a given organ and causes damage.

Diseases that are inflammatory based would be top targets for the diagnostic, and treatments that are anti-inflammatory in nature would be a primary use for testing treatment efficacy and separating treatment responders from non-responders.

Secondarily, very many diseases result in damage to an organ and inflammation follows. This is, for example the case with Stroke and epilepsy and Parkinson's Disease, though there are very many diseases for which secondary inflammation and even chronic inflammation play a part.

Therefore, this invention would also be valuable for diseases of this nature, to follow the damage caused, the expansion of the damage and a resolution of the disease, as would accompany a reduction in the biomarker. Additionally, this could be coupled to treatment efficacy on the underlying disease, since inflammation is a secondary marker of disease severity, and also opens value for separation of treatment responders and non-responders.

It is further contemplated that the biomarkers may be used in a method to distinguish between organ systems. This will be of value to determine where in the body the inflammation is coming from. For example, inflammation from a Lupus patient can be from the kidney or skin.

The assay for detection of the biomarkers is antibody-based, wherein the antibody binds to a specific epitope of the biomarker. A labelled secondary antibody is used to visualize the antibody-antigen complex for example via chemiluminescence detection system based on HRP enzymatic activity. As an alternative, fluorescent linking which is more stable and consistent may be used.

The method for identification of the GLX-biomarkers may be immunostrips. Immunostrips are immunosensors where the recognition agent is an antibody that binds to the analyte with detection by reflectance or fluorescence spectrophotometry.

Detection systems suitable for detecting the biomarkers of the present invention includes but is not limited to immunoassays based on lanthanides, lectins, GAG-binding molecules (table 2), Alcian Blue, Toluidine blue or dimethylmethylene blue.

**Table 2:**

| **GAG-Binding Proteins** | | |
|---|---|---|
| **Fibroblast growth factors (FGF) - Important possibility** | | |
| **Antithrombin** | | |
| Enzymes | | GAG biosynthetic enzymes, thrombin and coagulation factors (proteases), complement proteins (esterases), extracellular superoxide dismutase, lipases |
| Enzyme inhibitors | | antithrombin III, heparin cofactor II, secretory leukocyte proteinase inhibitor, C1-esterase inhibitor |
| Cell adhesion proteins | | P-selectin, L-selectin, some integrins |
| Extracellular matrix proteins | | laminin, fibronectin, collagens, thrombospondin, vitronectin, tenascin |
| Chemokines | | platelet factor IV, y- and β-interferons, interleukins, CXCL8, CXCL12, CCL2, CCL5 and CCL7 |
| Growth factors | | fibroblast growth factors, hepatocyte growth factor, vascular endothelial growth factor, insulin-like growth factor-binding proteins, TGF-p-binding proteins |
| Morphogens | | hedgehogs, TGF-β family members, wnts |
| Guidance factors | | slits, ROBO receptors, neuropilins |
| Tyrosine-kinase growth factor receptors and coreceptors | | fibroblast growth factor receptors, vascular endothelium growth factor receptor, RAGE, RPTPs |
| Lipid-binding proteins | | apolipoproteins B, E, and A-V, lipoprotein lipase, hepatic lipase, annexins |
| Plaque proteins | | prion proteins, amyloid proteins |
| Nuclear proteins | | histones, transcription factors |
| Pathogen surface proteins | | malaria circumsporozoite protein |
| Viral envelope proteins | | herpes simplex virus, dengue virus, Zika virus, human immunodeficiency virus, hepatitis C virus, VCP |

Other methods for identification if the GLX-biomarkers may be mass spectrometry (MS) such as MALDI-MS, LC-MS, HPLC-MS or Liquid chromatography-electrospray ionization-tandem mass spectrometry.

The platform for detecting one or more biomarkers in one sample (so called multiplexing) may be in a chip-based device.

A kit will comprise a panel of all GLX markers deemed valuable as biomarkers for a specific disease. These will be detecting molecules (e.g. antibodies) that are chemically attached to a surface (e.g. a plate or a tube) which will specifically bind each individual GLX marker. This kit will take one biological sample and test for all GLX markers in one run, comparing to reference values. This is a multiplex assay that will provide a readout of each marker compared to reference and determine whether a given disease is active or, in the case of treatment response and/or autoimmune disease, in remission.

Chip-based technologies combining these elements where the device will send the signal generated through a bluetooth, remote sensoring technology.

The chip system may be a microfluidics chip.

### Definitions

Prior to discussing the present invention in further details, the following terms and conventions will first be defined:

### Immune-mediated inflammatory disease

Immune-mediated inflammatory disease (IMID) is any of a group of conditions or diseases which are characterized by common inflammatory pathways leading to inflammation, and which may result from, or be triggered by, a dysregulation of the normal immune response. All IMIDs can cause end organ damage, and are associated with increased morbidity and/or mortality. An IMID could be any disease from the list given in table 1.

### GLX molecules

The "GLX" or "glycocalyx" is the carbohydrate-rich outer part of the cell surface of the majority of cells in the body, including the luminal endothelium. This layer is the first interaction between the blood and the vessel wall, both throughout the body and at the blood-brain barrier (BBB) junction. As described in here, shedding of the GLX may be an early stage predictor of MS, disease severity of MS, and treatment efficacy of MS. Examples of GLX molecules are Glycosaminoglycans 5 (GAGs) and Proteoglycans (PGs). These may be brain derived. Thus, the term "GLX-related" is to be understood as molecules associated with (or has been associated with) the GLX structure. Phrased in another way, the "GLX-related" may be understood as molecules originating from the GLX structure.

### Glycosaminoglycans

Glycosaminoglycan (GAGs) or "mucopolysaccharides" are long unbranched polysaccharides consisting of a repeating disaccharide unit. The repeating unit (except for keratan) consists of an amino sugar (N-acetylglucosamine or N-acetylgalactosamine) along with an uronic sugar (glucuronic acid or iduronic acid) or galactose. Examples of Glycosaminoglycan (GAGs) forming part of the present invention are:
- Chondroitin sulfate: Chondroitin sulfate (CS) is a sulfated glycosaminoglycan (GAG) composed of a chain of alternating sugars (N20 acetylgalactosamine and glucuronic acid). It is usually found attached to proteins as part of a proteoglycan.
- Hyaluronic acid: Hyaluronic acid (HA) also called hyaluronan, is an anionic, nonsulfated glycosaminoglycan (GAG) distributed widely throughout connective, epithelial, and neural tissues. It is unique among glycosaminoglycans in that it is non-sulfated, forms in the plasma membrane instead of the Golgi apparatus, and can be very large, with its molecular weight often reaching the millions.
- Heparan sulfate: Heparan sulfate (HS) is a linear polysaccharide found in all animal tissues. It occurs as a proteoglycan (HSPG) in which two or three HS chains are attached in close proximity to cell surface or extracellular matrix proteins.

### Proteoglycans

Proteoglycans (PGs) are proteins that are heavily glycosylated. The basic proteoglycan unit consists of a "core protein" with one or more covalently attached glycosaminoglycan (GAG) chain(s). Examples of proteoglycans forming part of the present invention are:
- Syndecans: Syndecans are single transmembrane domain proteoglycans that are thought to act as co-receptors, especially for G protein-coupled receptors. These core proteins carry heparan sulfate (HS) and chondroitin sulfate (CS) chains. The syndecan protein family has four members; Syndecan 1-4.
- Glypicans: Glypicans (GPC) constitute one of the two major families of heparan sulfate proteoglycans, with the other major family being syndecans. Six glypicans have been identified in mammals, and are referred to as GPC1-GPC6.
- Biglycan: Biglycan is a small leucine-rich repeat proteoglycan (SLRP) which is found in a variety of extracellular matrix tissues.

### Reference level

In the context of the present invention, the term "reference level" relates to a standard in relation to a quantity, which other values or characteristics can be compared to. In one embodiment of the present invention, it is possible to determine a reference level by investigating the abundance of one or more of the biomarkers according to the invention in biological or blood samples from healthy subjects. By applying different statistical means, such as multivariate analysis, one or more reference levels can be calculated. Based on these results, a cut-off may be obtained that shows the relationship between the level(s) detected and patients at risk. The cut-off can thereby be used to determine the amount of the one or more biomarkers, which corresponds to for instance an increased risk of stroke.

### Risk Assessment

The present inventors have successfully developed a new method to predict the risk for developing multiple sclerosis (MS) for a subject. The results presented in the examples show that the described biomarkers (alone or in combination) appear to be efficient biomarkers for determining whether a patient has an increased risk of developing stroke.

To determine whether a patient has an increased risk of developing stroke or having an incident of acute stroke a cut-off must be established. This cut-off may be established by the laboratory, the physician or on a case-by-case basis for each patient. The cut-off level could be established using a number of methods, including: multivariate statistical tests (such as partial least squares discriminant analysis (PLS-DA), random forest, support vector machine, etc.), percentiles, mean plus or minus standard deviation(s); median value; fold changes. The multivariate discriminant analysis and other risk assessments can be performed on the free or commercially available computer statistical packages (SAS, SPSS, Matlab, R, etc.) or other statistical software packages or screening software known to those skilled in the art.

As obvious to one skilled in the art, in any of the embodiments discussed above, changing the risk cut-off level could change the results of the discriminant analysis for each subject. Statistics enables evaluation of the significance of each level. Commonly used statistical tests applied to a data set include t-test, f-test or even more advanced tests and methods of comparing data. Using such a test or method enables the determination of whether two or more samples are significantly different or not.

The significance may be determined by the standard statistical methodology known by the person skilled in the art. The chosen reference level may be changed depending on the mammal/subject for which the test is applied. Preferably, the subject according to the invention is a human subject, such as a subject considered at risk of having or developing stroke.

The chosen reference level may be changed if desired to give a different specificity or sensitivity as known in the art. Sensitivity and specificity are widely used statistics to describe and quantify how good and reliable a biomarker or a diagnostic test is. Sensitivity evaluates how good a biomarker or a diagnostic test is at detecting a disease, while specificity estimates how likely an individual (i.e. control, patient without disease) can be correctly identified as not sick.

Several terms are used along with the description of sensitivity and specificity; true positives (TP), true negatives (TN), false negatives (FN) and false positives. If a disease is proven to be present in a sick patient, and the diagnostic test confirms the presence of disease, the result of the diagnostic test is considered to be TP. If a disease is not present in an individual (i.e. control, patient without disease), and the diagnostic test confirms the absence of disease, the test result is TN. If the diagnostic test indicates the presence of disease in an individual with no such disease, the test result is FP. Finally, if the diagnostic test indicates no presence of disease in a patient with disease, the test result is FN.

### Sensitivity

Sensitivity = TP/ (TP + FN) = number of true positive assessments/ number of all samples from patients with disease. As used herein, the sensitivity refers to the measures of the proportion of actual positives, which are correctly identified as such - in analogy with a diagnostic test, i.e. the percentage of people having PaO2 below normal who are identified as having PaO2 below normal.

### Specificity

Specificity= TN/ (TN+ FP) = number of true negative assessments/ number of all samples from controls. As used herein, the specificity refers to measures of the proportion of negatives, which are correctly identified. The relationship between both sensitivity and specificity can be assessed by the ROC curve. This graphical representation helps to decide the optimal model through determining the best threshold -or cut-off for a diagnostic test or a biomarker candidate.

As will be generally understood by those skilled in the art, methods for screening are processes of decision-making and therefore the chosen specificity and sensitivity depend on what is considered to be the optimal outcome by a given assay.

It would be obvious for a person skilled in the art that it may be advantageous to select a higher sensitivity at the expense of lower specificity in most cases, to identify as many patients with disease risk as possible.
In a preferred embodiment, the invention relates to a method with a high specificity, such as at least 70%, such as at least 80%, such as at least 90%, such as at least 95%, such as 100%. In another preferred embodiment, the invention relates to a method with a high sensitivity, such as at least 80%, such as at least 90%, such as 100%.

### Examples

### Example 1: Blood from Human Stroke patients

To show that biomarkers of the GLX structure are detectable in blood samples and that these biomarkers increase in response to disease and inflammation, blood samples obtained from patients 1 day, 10 days, and 90 days after an acute stroke incident were tested and compared to the level of the GLX markers in blood samples from healthy age-matched individuals (the reference sample is indicated with the abbreviations for the GLX marker on the x-axis in the fig. 1 a) to h)).

### Materials and Methods

### Plasma Dot Blotting

Antibody-based dot blots were used to assess GLX markers longitudinally. Two µl of plasma was dotted in duplicate on a cationic nitrocellulose membrane (Hybond N+, Amersham, GE Healthcare, Brondby, Denmark) and allowed to dry. The membrane was incubated for 60 minutes at room temperature in blocking buffer: 5% skim milk powder (Sigma-Aldrich) in tris-buffered saline + 0.05% Tween20 (TBS-T; Sigma-Aldrich). The membrane was incubated thereafter with primary antibodies at their respective dilutions overnight, at 4°C. Membranes were thereafter washed in TBS-T and incubated with secondary antibodies conjugated to horseradish peroxidase, diluted at respective dilutions in blocking buffer, and raised against the source of the primary for 60 mins. Membranes were washed thoroughly with TBS-T and finally, in TBS. Membranes were visualized with Supersignal West femto luminescent substrate and Chemidoc XRS CCD camera (Bio-Rad Laboratories). Chemiluminescence was quantified with densitometry after normalizing to background with ImageJ software.

Primary antibodies: Heparan sulfate (HS) (1:1500, MAB2040, Millipore), Syndecan-1 (clone MI15 (cat no M722801-2, Dako/Agilent, USA), Syndecan-4 (clone 5G9, (cat no SC-12766, Santa Cruz, USA)). Chondroitin sulfate (CS) (1:1000, CS-56, cat no C8035, Sigma-Aldrich, Brøndby, Denmark), Syndecan-3 (1:1000, cat no AF3539, R&D Systems, UK). HA (cat no 5029-9990, Bio-Rad, USA), glypican-1 (cat no AF4519, R&D Systems), CD44 (1:200, DAKO, Glostrup, Denmark._{[BWDV1]}

Secondary antibodies: HRP-conjugated: anti-rabbit (1:2000), anti-rat (1:4000), anti-mouse (1:3000) (DAKO, Glostrup, DK).

Visualisation and optical density analysis is performed as above with a secondary antibody-HRP complex and chemiluminescence (femtogram resolution). Tests were performed for the following biomarkers:
Proteoglycans:
   Syndecan-1 (fig. 1 f))
   Syndecan-3 (fig. 1 a))
   Syndecan-4 (fig. 1 e))
   Glypican-1 (fig. 1g))
   CD44 (fig. 1 c))
Glycosaminoglycans:
   Chondroitin Sulfates (CS) (fig. 1b))
   Heparan Sulfates (HS) (fig. 1 d))
   Hyaluronic Acids (HA) (fig. 1 h))

### Data analysis:

Data sets were tested for normality (Shapiro-Wilk) and equal variance before statistical analyses were performed (One way ANOVA). Data that was non-normal was log-transformed and re-tested for normality and equal variance. Data sets were then analyzed with parametric or non-parametric statistics based on normality after transformation. A P-value of <0.05 was reported as statistically significantly different. Data are presented as mean ± S.E.M for normal data and median ± interquartile range for non-normal data.

### Results

As seen in figure 1 a) to h), all GLX markers are detectable above background in all patient and healthy control samples. All GLX markers display a unique signature both between patients and healthy controls and within each patient over the timescale. As seen in Fig. 1, Syndecan-3, Chondroitin Sulfate (CS), CD44 and Heparan Sulfate (HS) are particularly effective in separating healthy control samples from Stroke patient sample at the 7 day time interval.

### Conclusion

In this set of experiments, it is shown that GLX components across different classes, are elevated and variable between Stroke patients and over time within each patient and thus should be considered as potential biomarkers of disease, disease severity, and treatment response. The classes of GLX that have shown to be of substantial relevance are glycosaminoglycans (GAGs) and proteoglycans (PGs).

When taken together, in here is provided evidence for the identification of GLX components consisting of: GAGs: Chondroitin Sulfates, Hyaluronic Acids, and Heparan Sulfates and PGs: Syndecans, and Glypicans; in biological samples. These represent the vast majority of the GLX structure and are all identified as being indicative of being biomarkers for Stroke disease.

## Claims

1. An *ex vivo* method of diagnosing and/or prognosticating at least one immune-mediated inflammatory disease (IMID) in a subject, said method comprising the steps of
a. performing *in vitro* measurement of the level of one or more biomarkers selected from the group consisting of glycosaminoglycans (GAGs) and proteoglycans (PGs) of the glycocalyx in a first biological sample from the subject;
b. optionally, performing *in vitro* measurement of the level of the one or more biomarkers in a second or further biological samples from the subject obtained at a later time point than said first biological sample.
c. comparing the level of said one or more biomarkers to the level of two or more corresponding references; and
d. using the measured value to evaluate the state of the subject.

2. The *ex vivo* method according to claim 1, wherein the state of the subject evaluated in step d. is determined as at risk of developing and/or having IMID, if said level of two or more biomarkers are above the corresponding one or more reference levels, and determined as not at risk of developing or having IMID, if said one or more levels are equal to or below the one or more corresponding reference levels.

3. The *ex vivo* method according to claim 1 or 2, wherein the level of the one or more biomarkers is significantly greater than the corresponding reference value with a P-value of at least <0.05.

4. The ex vivo method according to any of claims 1 to 3, wherein the biological sample is a blood sample, such as whole blood, plasma, serum or cerebrospinal fluid.

5. The ex vivo method according to any of claims 1 to 4, wherein the glycosaminoglycan (GAG) is selected from one or more of the group consisting of chondroitin sulfate (CS), heparan sulfate (HS), hyaluronic acid (HA), dermatan sulfate (DS), keratin sulfate (KS).

6. The ex vivo method according to any of the claims 1 to 5, wherein the proteoglycan (PG) is selected from one or more of the group consisting of syndecans, glypicans, and biglycans.

7. The ex vivo method according to any of the claims 1 to 6, wherein the syndecan is selected from one or more of the group consisting of syndecan-1, syndecan-2, syndecan-3 and syndecan-4.

8. The ex vivo method according to any of the claims 1 to 7, wherein the glypican is selected from one or more of the group consisting of, glypican-1, glypican-2, glypican-3, glypican-4, glypican-5 and glypican-6.

9. The ex vivo method according to any of the claims 1 to 8, wherein the proteoglycan (PG) is selected from one or more of the group consisting of biglycan, perlecan, mimecan, decorin and versican.

10. The *ex vivo* method according to any one of the preceding claims, wherein the biomarkers are the combination of Syndecan-3, Chondroitin Sulfate (CS), CD44, Heparan Sulfate (HS), Syndecan-4, Syndecan-1, Glypican-1 and Hyaluronic Acid (HA).

11. The *ex vivo* method according to any one of the preceding claims, wherein the at least one IMID is an autoimmune disease, a neuroinflammatory disease or an organ system disease.

12. The *ex vivo* method according to any one of the preceding claims, wherein the at least one IMID is a stroke.

13. The *ex vivo* method according to any of the preceding claims, wherein the amount of biological sample tested is higher than or equal to 0.25 µL.

14. The *ex vivo* method according to any of the preceding claims wherein at least two biomarkersare measured .

15. A substance for use in a method of treating a condition in an individual known to have at least one or more biomarkers selected from the group consisting of glycosaminoglycans (GAGs) and proteoglycans (PGs) of the glycocalyx, wherein the biomarkers are at level above the corresponding one or more reference levels, wherein the substance and condition, respectively, are selected from:
| **Drug** | **IMID** |
|---|---|
| Biologics | Autoimmune Disease |
| Non-steroidal anti-inflammatory drugs | |
| Glucocorticoids | |
| Disease-modifying antirheumatic drugs | |
| Biosimilars | |
| Vasodilators | Stroke |
| Statins | |
| Anticonvulsants | |
| ACE Inhibitors | |
| Cholinesterase inhibitors | Alzheimer's Disease |
| Levodopa | Parkinson's Disease |
| Carbidopa | |
| Carbidopa-levodopa | |
| Dopamine agonists | |
| MAO B inhibitors | |
| Catechol O-methyltransferase (COMT) inhibitors | |
| Anticholinergics | |
| Amantadine | |

16. A substance for use in a method of treating a condition according to claim 15, wherein the substance and condition, respectively, are selected from:
| **Drug** | **IMID** |
|---|---|
| Immunosuppressant Drugs | Lupus |
| Cyclophosphamide | |
| Azathioprine | |
| Hydroxychloroquine | |
| Methotrexate | |
| Ibuprofen | |
| Naproxen | |
| Sulindac | |
| misoprostol | |
| Prednisolone | |
| methylprednisolone | |
| butesonide | |
| chloroquine | |
| dapsone | |
| chlorambucil | |
| mycophenolate mofetil | |
| Belimumab | |
| Rituximab | |
| dehydroepiandrosterone | |
| Immunosuppressant Drugs | Rheumatoid Arthritis |
| Cyclophosphamide | |
| Azathioprine | |
| Belimumab | |
| Hydroxychloroquine | |
| Rituximab | |
| Ibuprofen | |
| Etanercept | |
| methotrexate | |
| sulfasalazine | |
| leflunomide | |
| Infliximab | |
| prednisolone | |
| methylprednisolone | |
| butesonide | |
| Immunosuppressant Drugs | Inflammatory Bowel Disease |
| Mesalazine | |
| prednisolone | |
| methylprednisolone | |
| butesonide | |
| Azathioprine | |
| methotrexate | |
| cyclosporin | |
| tacrolimus | |
| adalimumab | |
| Infliximab | |
| certolizumab pegol | |
| Ornidazole | |
| Metronidazole | |
| clarithromycin | |
| rifaximin ciprofloxacin | |
| infliximab | Celiac Disease |
| infliximab-abda | |
| infliximab-dyyb | |
| balsalazide | Crohn's Disease |
| Mesalamine | |
| olsalazine | |
| Sulfasalazine | |
| Tacrolimus | |
| prednisolone | |
| methylprednisolone | |
| Butesonide | |
| Azathioprine | |
| methotrexate | |
| Cyclosporin | |
| Golimumab | |
| adalimumab-adbm | |
| Adalimumab | |
| Infliximab | |
| infliximab-abda | |
| infliximab-dyyb | |
| vedolizumab | |
| balsalazide | Ulcerative Colitis |
| Mesalamine | |
| olsalazine | |
| Sulfasalazine | |
| prednisolone | |
| methylprednisolone | |
| Butesonide | |
| Azathioprine | |
| methotrexate | |
| cyclosporin | |
| golimumab | |
| adalimumab-adbm | |
| adalimumab | |
| Infliximab | |
| infliximab-abda | |
| infliximab-dyyb | |
| vedolizumab | |
| Calcipotriene | Psoriasis |
| Acitretin | |
| Methoxsalen | |
| Trioxsalen | |
| Adalimumab | |
| Etanercept | |
| Infliximab | |
| Secukinumab | |
| Ixekizumab | |
| Apremilast | |
| Methotrexate | |
| Cyclosporin | |
| Atorvastatin | Stroke |
| fluvastatin | |
| lovastatin | |
| Pitavastatin | |
| Pravastatin | |
| Rosuvastatin | |
| simvastatin | |
| Coumadin | |
| Jantoven | |
| Marfarin | |
| Clopidogrel | |
| Tissue plasminogen activator | |
| angiotensin-converting enzyme inhibitors | |
| beta-blockers | |
| calcium channel blockers | |
| Galantamine | Alzheimer's Disease |
| Donepezil | |
| Rivastigmine | |
| Memantine | |
| Citalopram | |
| Mirtazapine | |
| Sertraline | |
| Bupropion | |
| Duloxetine | |
| Imipramine | |

17. A substance selected from the group consisting of atorvastatin, fluvastatin, lovastatin, pitavastatin, pravastatin, rosuvastatin, simvastatin, Coumadin, Jantoven, Marfarin, clopidogrel, Tissue plasminogen activator, angiotensin-converting enzyme inhibitors, beta-blockers, calcium channel blockers for use in a method of treating a stroke in an individual known to have at least one or more biomarkers selected from the group consisting of glycosaminoglycans (GAGs) and proteoglycans (PGs) of the glycocalyx.

18. A kit comprising
- binding agents for at least two biomarkers selected from the group consisting of glycosaminoglycans (GAGs) and proteoglycans (PGs) of the glycocalyx; and
- optionally instructions for using the binding agents in the evaluation of IMID.

19. A kit comprising
- binding agents for Syndecan-3, Chondroitin Sulfate (CS), CD44, Heparan Sulfate (HS), Syndecan-4, Syndecan-1, Glypican-1 and Hyaluronic Acid (HA), respectively; and
- instructions for using the binding agents in the evaluation of stroke.

20. A microfluidic detection chip (1) for the detection of an IMID infection in a patient said chip comprising one or more separation channels (2) which are coated with one or more binding agents for the biomarkers selected from the group consisting of glycosaminoglycans (GAGs) and proteoglycans (PGs) of the glycocalyx.

21. A microfluidic detection chip according to claim 20 for the detection of at least one IMID infection in a patient, said chip (1) comprising one or more layers (3) in which a plurality of separation channels (2) are disposed in each layer; at least one sample inlet port (4) which is in fluid communication with the separation channels (2) and into which a biological sample is introduced; wherein one or more of the separation channels (2) have a plurality of three-dimensional (3D) separation zones (5); with an intermittent partition zone wherein the chip is optic.

22. The kit according to claims 18 or 19 further comprising the microfluidic chip according to claim 20 or 21.
